# EUROPEAN PATENT APPLICATION

(11) **EP 3 944 865 A1**
(43) Date of publication of application: **02.02.2022**
(21) Application number: 21181582.4
(22) Date of filing: 24.06.2021
(51) Int. Cl.: A61L 2/20, A61L 2/24, A61L 9/015, A61L 9/12, B60H 3/00

(54) **DEVICE FOR SANITIZING A VEHICLE**

(30) Priority: 31.07.2020 IT 202000018877
(71) Applicant: Texa SpA, 31050 Monastier di Treviso (TV) (IT)
(72) Inventor: VIANELLO, Bruno, 31050 MONASTIER DI TREVISO (TV) (IT)
(74) Representative: Burchielli, Riccardo

(57) **Abstract**

A sanitizing device for a vehicle (20) which, when in use, is adapted to deliver chemical agents and/or gases for sanitizing the interior of a vehicle (10). Additionally, the sanitizing device (20) integrally comprises a pressure sensor (30) adapted to monitor the pressure inside the vehicle (10) when the sanitizing device (20) is in use.

In particular, when the door (11) is opened, the sensor (30) detects a pressure gradient, thus interrupting the delivery of the chemical agents and/or gases inside the vehicle (10).

## Description

This invention relates generally to a sanitizing device of a vehicle.

In particular, this invention relates to a safety/emergency system which intervenes if there is an incorrect use of the sanitizing device and in general, if there is a need to do so.

Vehicles that are used every day, regardless of their type, are highly exposed to bacterial contamination and viruses such as, for example the current case of COVID-19, more commonly known as 'Coronavirus'.

In particular, vehicles can be used for a wide variety of activities and jobs, just think of passenger vehicles such as, for example coaches, taxis and ambulances.

A contamination phenomenon that often occurs, particularly in these types of vehicles, is cross-contamination, i.e. the direct or indirect passage of microbes and pathogens through a source such as in this case, the passenger compartment of the vehicle, through the likely contact of the hands of several people.

There are currently several types of vehicle sanitizing systems, the two main ones providing the introduction of chemical agents or gases such as ozone or gels into the vehicle through a nebulisation system, which deposits them on the internal surfaces of the vehicle, sterilising them, thus reducing the bacterial and/or viral load.

In detail, in the event gases are used, it must be removed after the desired sanitizing effect has been achieved so as not to create harmful effects on people who will use the vehicle after sanitization.

In fact, the sanitization operation is not normally carried out with people inside the vehicle, as the sanitizing substances are often toxic to humans in the event of inhalation, ingestion or contact with the mucous membranes or eyes.

One of the main problems with the sanitizing systems used today is that if a user enters the vehicle while a sanitization cycle is in progress, without having previously stopped the sanitizing device, they could expose themselves and possibly other people to the action of the gas, as in the case of ozone, thus creating a dangerous situation.

For the reasons introduced above, it is necessary to use a safety system for sanitizing devices of a vehicle, which intervenes in the event of incorrect use of the sanitizing device itself, for example if the vehicle is entered during ozonization.

The aim of this invention is therefore to make a sanitizing device for a vehicle, which in the event of an emergency, guarantees the safety of the users during the use of the same.

A further aim of this invention is to make a sanitizing device for a vehicle which is easier and faster to use than the methods used to date.

Last but not least, it is an aim of this invention to make a sanitizing device for a vehicle which is more efficient and safer than the aforementioned known art and which overcomes its limitations.

These and other aims are achieved by a sanitizing device for a vehicle according to appended claim 1; other detailed features of the sanitizing device for a vehicle are set forth in the dependent claims.

Advantageously, unlike the solutions described and proposed so far, the sanitizing device for a vehicle according to this invention does not only sanitize the passenger compartment of the vehicle, but also guarantees the safety of the user by controlling emergency situations, in particular, by stopping the production of sanitizing gases inside the vehicle when necessary.

The above-mentioned aims and advantages will be more apparent from the following description of a preferred embodiment of the sanitizing device for a vehicle, which is the object of this invention, provided by way of non-limiting example, and the accompanying drawings, also provided by way of non-limiting example, wherein:
- Figure 1 is a perspective view of the sanitizing device for a vehicle according to the invention;
- Figure 2A is a top view of the sanitizing device according to the invention in Figure 1, placed inside the vehicle;
- Figure 2B is a top view of the sanitizing device for a vehicle, the object of this invention, when in use.

With reference to the mentioned figures, the vehicle on which the sanitizing device, the object of this invention, is installed, is generally indicated by 10, and at least one door of the vehicle 10 for access to the passenger compartment thereof is indicated by 11.

Numerical reference 20 indicates a sanitizing device adapted to be installed and/or placed inside the passenger compartment of the vehicle 10, for example by means of a means 22 for transportation, in particular, a handle. Advantageously, the sanitizing device 20 in preferred embodiments has a fan 21 adapted to deliver and favour the diffusion of a sanitizing agent inside the vehicle 10 itself.

Furthermore, the aforementioned sanitizing device 20 is adapted to deliver 10 different types of sanitizing chemical agents or gases inside the vehicle, depending on the sanitizing operation being carried out. Advantageously, one of the gases most commonly used by the sanitizing device 20, the object of this invention, is ozone; at present, ozone is a gas whose peculiarities with respect to its disinfecting power, and therefore its sanitizing power, are well known and in addition to being recognised as usable in the circumstances described above, it has also been recognised as usable by the legislature in force (in fact, protocol no. 24482 of 31 July 1996 of the Ministry of Health recognised the ozone sanitizing system as a valid method for the sterilisation of contaminated environments, with a series of scientific validations).

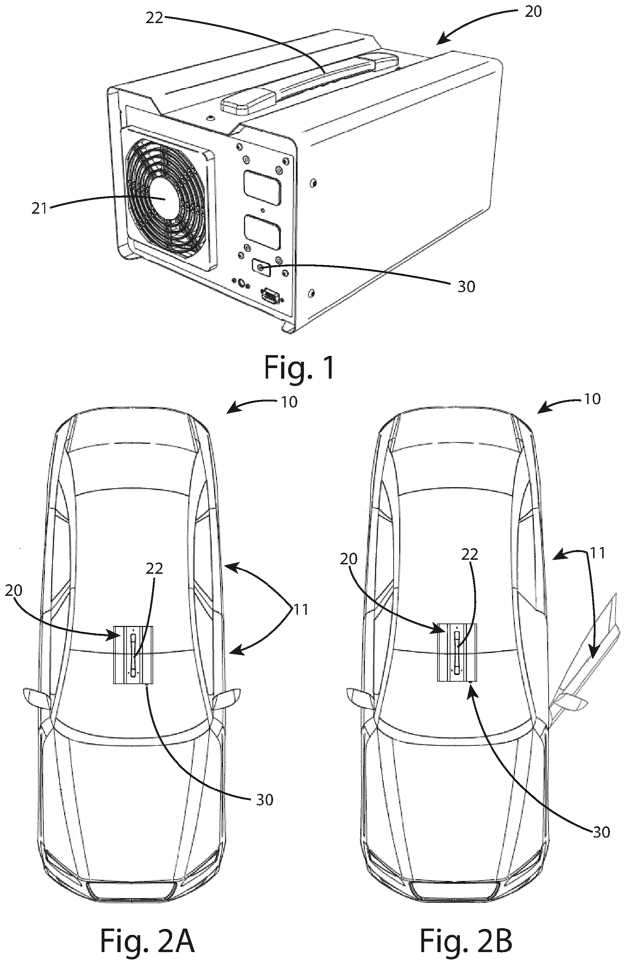

As can be seen in Figure 1, the aforementioned sanitizing device 20 comprises, integrated thereon, a pressure sensor 30 adapted to monitor the pressure inside the passenger compartment of the vehicle 10 (Figure 2A).

In preferred non-limiting embodiments, the sanitizing device 20 bases its operation on controlling the ozone saturation of the air in the passenger compartment of the vehicle 10 and its subsequent abatement in this case using a controlled sanitizing cycle based on a time established according to the geometric characteristics (e.g. size, volume, etc.) of the vehicle 10 to be sanitized.

Advantageously, in the event at least one door 11 of the vehicle 10 is opened, the pressure sensor 30 detects a pressure variation, i.e. a pressure gradient, considered as an indication of an operating error; this variation may arise, for example when the user enters the vehicle 10 during sanitizing before stopping the sanitizing device 20 itself.

Advantageously, if the sanitizing device 20 continues generating the sanitizing substance despite the door 11 of the vehicle 10 being opened, the sensor 30 interrupts the delivery of the sanitizing substance by stopping the operation of the sanitizing device 20 so as to avoid the saturation of the air in the passenger compartment of the vehicle 10 itself.

By virtue of the foregoing, it is understood how the safety stopping system for sanitizing devices of a vehicle, which is the object of this invention, achieves the above-mentioned aims and advantages.

Lastly, it is clear that numerous other variants might be made to the safety blocking system according to the invention, without thereby departing from the scope of protection of the invention expressed in the accompanying claims, and it is also clear that in the practical actuation of the invention, the materials, the shapes and the dimensions of the illustrated details can be of any type and can be replaced, according to requirements, by other technically equivalent elements.

## Claims

1. Sanitizing device of a vehicle (10) suitable to supply, when in use, chemical agents and/or gases for sanitizing the interior of said vehicle (10); said vehicle (10) comprising at least one door (11) which is closed when said sanitizing device (20) is in use, **characterized in that** said sanitizing device (20) comprises a pressure sensor (30) adapted to monitor the pressure inside said vehicle (10) when said sanitizing device (20) is in use, so that when said at least a door (11) is open, said sensor (30) detects a gradient of said internal pressure and interrupts the delivery of said chemical agents and/or gases by said sanitizing device (20).

2. Sanitizing device (20) as in claim 1 **characterized in that** said device (20) is installed and/or placed inside said vehicle (10).

3. Sanitizing device (20) as at least one of the preceding claims, **characterized in that** said chemical agents and/or gases supplied by said sanitizing device (20) comprise ozone.

4. Sanitizing device (20) as at least one of the preceding claims, **characterized in that** said device (20) comprises a fan (21) adapted to deliver and to favor the diffusion of said chemical agents and/or gases inside said vehicle (10).

5. Sanitizing device (20) as at least one of the preceding claims, **characterized in that** said device (20) comprises a means for transportation (22), in particular a handle.

6. Sanitizing device (20) as at least one of the preceding claims, **characterized in that** said device (20) is suitable to control the saturation of the air inside said vehicle (10) by means of a timed and pre-established sanitization cycle based on the geometric characteristics of said vehicle (10).
